# EUROPEAN PATENT APPLICATION

(11) **EP 4 813 309 A1**
(43) Date of publication of application: **30.09.2026**
(21) Application number: 24893873.0
(22) Date of filing: 03.10.2024
(51) Int. Cl.: A61B 6/08, A61B 6/00

(54) **X-RAY IMAGING SYSTEM AND IMAGING UNIT**

(30) Priority: 20.11.2023 JP 2023197007
(71) Applicant: SHIMADZU CORPORATION, Kyoto-shi, Kyoto 604-8511 (JP)
(72) Inventor: NAKATSU, Kohei, Kyoto-shi, Kyoto 604-8511 (JP); FURUHASHI, Naoya, Kyoto-shi, Kyoto 604-8511 (JP); TAKEDA, Ryo, Kyoto-shi, Kyoto 604-8511 (JP); OKUMURA, Hiroshi, Kyoto-shi, Kyoto 604-8511 (JP)
(74) Representative: Müller-Boré & Partner Patentanwälte PartG mbB
(86) International application number: PCT/JP2024/035448
(87) International publication number: WO 2025/109879

(57) **Abstract**

This X-ray imaging system (100) comprises: an X-ray irradiation unit (10); an X-ray detection unit (20) that detects X-rays; an optical imaging unit (31) that captures an optical image (70) of a subject (101); a display unit (41); and a control unit (32) configured to cause the display unit (41) to display, superimposed on the optical image (70), a position reference image (90) being arranged inside the X-ray detection unit (20) and indicating a position relative to a center of the X-ray detection unit (20).

## Description

### Technical Field

The present invention relates to an X-ray imaging system and an imaging unit.

### Background Art

Conventionally, an X-ray imaging system including an optical imaging unit is known. Such an X-ray imaging system is disclosed, for example, in International Publication No. 2022/271583.

The above-mentioned International Publication No. 2022/271583 describes a radiographic imaging system. In this radiographic imaging system, a camera is arranged on a tube head having an X-ray source. The camera is arranged toward a patient, and a video image of the patient is displayed on a display. A digital X-ray detector is arranged on a bed on which the patient lies. In the X-ray imaging system described in the above-mentioned International Publication No. 2022/271583, the position of the digital X-ray detector in the video image captured by the camera is detected, and a semi-transparent rectangular cursor is overlaid and displayed on the video image so as to indicate the position of the digital X-ray detector in the video image.

### Prior Art Documents

### Patent Documents

Patent Document 1: International Publication No. 2022/271583

### Summary of the Invention

### Problems to be Solved by the Invention

However, as in the radiographic imaging system (X-ray imaging system) of the above-mentioned International Publication No. 2022/271583, when the position of an X-ray detection unit that detects X-rays is indicated by a rectangle, it becomes difficult to confirm a specific position inside the rectangular region of a part to be subjected to X-ray imaging of the subject. That is, it becomes difficult to confirm exactly at which position inside the region indicated by the rectangular shape the part to be subjected to X-ray imaging of the subject is arranged. Therefore, it is desired to confirm the arrangement of the subject in the region where X-rays are detected.

The present invention has been made to solve the above-described problems, and one object of the present invention is to provide an X-ray imaging system and an imaging unit capable of confirming the arrangement of a subject in a region where X-rays are detected.

### Means for Solving the Problems

An X-ray imaging system according to a first aspect of the present invention includes: an X-ray irradiation unit including an X-ray tube; an X-ray detection unit configured to detect X-rays irradiated from the X-ray irradiation unit and transmitted through a subject; an optical imaging unit configured to capture an optical image of the subject; a display unit configured to display the optical image captured by the optical imaging unit; and a control unit configured to cause the display unit to display, superimposed on the optical image, a position reference image arranged inside the X-ray detection unit and indicating a position relative to a center of the X-ray detection unit, on the optical image.

An imaging unit according to a second aspect of the present invention includes: an optical imaging unit configured to capture an optical image of a subject; and a control unit configured to cause a display unit to display, superimposed on the optical image, a position reference image arranged, in the optical image, inside an X-ray detection unit configured to detect X-rays irradiated from an X-ray irradiation unit and transmitted through the subject, and indicating a position relative to a center of the X-ray detection unit.

### Effects of the Invention

As described above, the X-ray imaging system according to the first aspect and the imaging unit according to the second aspect include the control unit configured to cause the display unit to superimpose and display the position reference image, which is arranged inside the X-ray detection unit and indicates the position relative to the center of the X-ray detection unit, on the optical image. Accordingly, since the position reference image arranged inside the X-ray detection unit and indicating the position relative to the center of the X-ray detection unit is superimposed and displayed on the optical image, an operator can visually recognize the optical image on which the position reference image is superimposed, thereby confirming the position of the subject inside the X-ray detection unit based on the position reference image. As a result, the arrangement of the subject can be confirmed in the region where X-rays are detected.

### Brief Description of the Drawings

[FIG. 1] FIG. 1 is a schematic diagram showing an overall configuration of an X-ray imaging system according to one embodiment.
[FIG. 2] FIG. 2 is a schematic diagram showing a configuration of a holding unit according to one embodiment.
[FIG. 3] FIG. 3 is a block diagram showing the overall configuration of the X-ray imaging system according to one embodiment.
[FIG. 4] FIG. 4 is a schematic diagram showing a configuration of an optical image generated by an optical imaging control unit.
[FIG. 5] FIG. 5 is a schematic diagram for explaining superimposition of a detection unit region display and a position reference image on the optical image.
[FIG. 6] FIG. 6 is a schematic diagram for explaining changing the size of the position reference image.
[FIG. 7] FIG. 7 is a schematic diagram for explaining changing the number of displayed position reference displays.
[FIG. 8] FIG. 8 is a schematic diagram for explaining superimposition of an irradiation field region display on the optical image.
[FIG. 9] FIG. 9 is a schematic diagram for explaining changing the size of the irradiation field region display.
[FIG. 10] FIG. 10 is a schematic diagram showing an example of superimposition of the detection unit region display and the position reference image on the optical image in a palmar part of the subject.
[FIG. 11] FIG. 11 is a flowchart diagram for explaining control processing of an image display method by the X-ray imaging system.

### Mode for Carrying Out the Invention

Hereinafter, embodiments embodying the present invention will be described with reference to the drawings.

### (Configuration of X-ray Imaging System)

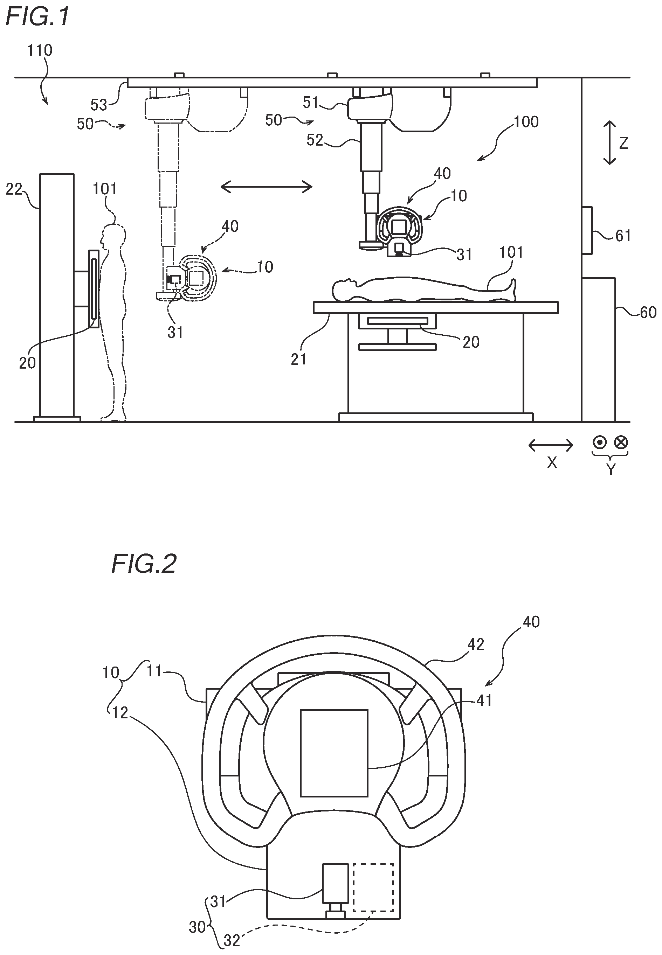

The configuration of an X-ray imaging system 100 according to one embodiment of the present invention will be described with reference to FIGS. 1 to 10.

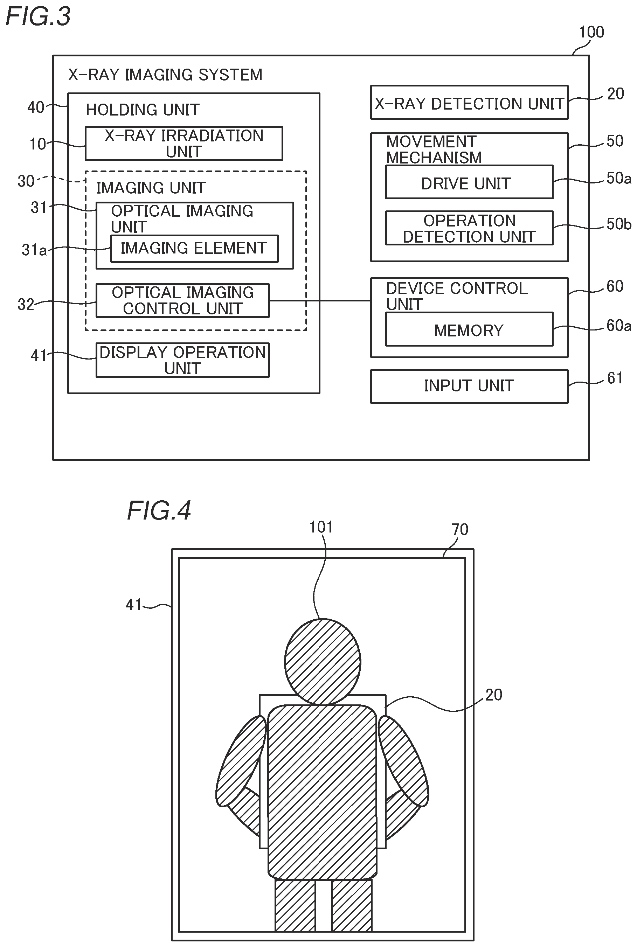

FIG. 1 shows an example of a ceiling-suspended X-ray imaging system 100. The X-ray imaging system 100 includes an X-ray irradiation unit 10, an X-ray detection unit 20, an optical imaging unit 31, a holding unit 40, a movement mechanism 50, a device control unit 60, and an input unit 61. The X-ray imaging system 100 is a system including a medical X-ray imaging apparatus, and is configured to perform X-ray imaging of a subject 101 to be imaged. In the X-ray imaging system 100, X-rays irradiated from the X-ray irradiation unit 10 are detected by the X-ray detection unit 20, whereby X-ray imaging of the subject 101 is performed. Further, in the X-ray imaging system 100, an optical image 70 (see FIG. 4) in which the appearance of the subject 101 is captured is imaged by the optical imaging unit 31. Further, in the X-ray imaging system 100, the X-ray irradiation unit 10, the X-ray detection unit 20, the optical imaging unit 31, the holding unit 40, and the movement mechanism 50 are installed in an imaging room 110, and the device control unit 60 and the input unit 61 are installed outside the imaging room 110.

In the ceiling-suspended X-ray imaging system 100, the holding unit 40 holding the X-ray irradiation unit 10 is held by the movement mechanism 50 arranged on the ceiling of the imaging room 110 so as to be suspended from the ceiling. The holding unit 40 is movably held in the imaging room 110 by the movement mechanism 50. The vertical (perpendicular) direction is defined as a Z direction, and two directions orthogonal to each other in the horizontal direction are defined as an X direction and a Y direction.

The X-ray imaging system 100 includes an imaging table 21 for performing imaging in a posture in which the subject 101 is lying down (decubitus position), and an imaging stand 22 for performing imaging in a posture in which the subject 101 is standing (standing position). The X-ray detection unit 20 is movably held by each of the imaging table 21 and the imaging stand 22. The X-ray detection unit 20 includes, for example, a flat panel detector (FPD). The X-ray detection unit 20 is configured to detect X-rays irradiated from the X-ray irradiation unit 10 and transmitted through the subject 101. The movement mechanism 50 can move the holding unit 40 at least between an imaging position in the decubitus position using the imaging table 21 (see solid line in FIG. 1) and an imaging position in the standing position using the imaging stand 22 (see two-dot chain line in FIG. 1).

In decubitus X-ray imaging, the holding unit 40 is arranged at a position vertically facing the X-ray detection unit 20 of the imaging table 21, X-ray imaging is performed on the subject 101 lying on the imaging table 21 between the vertically facing X-ray irradiation unit 10 and X-ray detection unit 20, and the subject 101 lying on the imaging table 21 is imaged (optically imaged) by the optical imaging unit 31. In standing X-ray imaging, the holding unit 40 is arranged at a position horizontally facing the X-ray detection unit 20 of the imaging stand 22, X-ray imaging is performed on the subject 101 standing in front of the imaging stand 22 between the horizontally facing X-ray irradiation unit 10 and X-ray detection unit 20, and the subject 101 standing in front of the imaging stand 22 is imaged by the optical imaging unit 31.

The movement mechanism 50 is configured to hold the holding unit 40 so as to be movable in a horizontal direction (X direction and Y direction) and a vertical direction (Z direction). The movement mechanism 50 includes a ceiling suspender 51 and a column unit 52. The movement mechanism 50 is supported by rails 53 provided on the ceiling of the imaging room 110. The ceiling suspender 51 is configured to be movable in the horizontal direction by the rails 53. The ceiling suspender 51 is configured to support the column unit 52. The column unit 52 is configured to support the holding unit 40. The column unit 52 is configured to be extendable and contractible in the vertical direction. The holding unit 40 is configured to be movable in the vertical direction by the column unit 52. In addition, the movement mechanism 50 moves the X-ray detection unit 20 arranged on each of the imaging table 21 and the imaging stand 22.

As shown in FIG. 2, the X-ray irradiation unit 10 includes an X-ray tube 11 and a collimator unit 12. The X-ray irradiation unit 10 is configured to irradiate the subject 101 with X-rays from the X-ray tube 11. The X-ray tube 11 is configured to irradiate X-rays by being applied with a predetermined voltage. The collimator unit 12 has a plurality of position-adjustable shielding plates (collimator leaves). The collimator unit 12 is configured to adjust an irradiation field of X-rays irradiated from the X-ray tube 11 by shielding a part of the X-rays from the X-ray tube 11. The collimator unit 12 is provided in the vicinity of the X-ray tube 11 in an X-ray irradiation direction of the X-ray tube 11.

The holding unit 40 includes a display operation unit 41 and a grip unit 42. The holding unit 40 is configured to be movable in the horizontal direction and the vertical direction via the movement mechanism 50 by manual movement or control by the device control unit 60. In the present embodiment, the optical imaging unit 31 is provided in the holding unit 40 together with the X-ray irradiation unit 10. Specifically, an imaging unit 30 composed of the optical imaging unit 31 and an optical imaging control unit 32 is arranged in the holding unit 40. The optical imaging control unit 32 and the display operation unit 41 are examples of a "control unit" and a "display unit" in the claims, respectively.

The display operation unit 41 includes, for example, a touch panel liquid crystal display. The display operation unit 41 is configured to function as an image display unit that displays the optical image 70 (see FIG. 4) captured by the optical imaging unit 31 and the like, and also to function as an operation unit that receives various operations input by an operator. The display operation unit 41 outputs a signal indicating the received input operation to the optical imaging control unit 32 and the device control unit 60.

The grip unit 42 is provided to be gripped by the operator when performing an operation of manually moving the holding unit 40. The grip unit 42 transmits an operation force of the operator to the holding unit 40.

The optical imaging unit 31 is provided on an outer side surface of the collimator unit 12. In the present embodiment, the optical imaging unit 31 is provided on an outer side surface of the collimator unit 12 on a longitudinal direction side of the imaging table 21 at an imaging position in the decubitus position. Also, the optical imaging unit 31 is provided on an outer side surface of the collimator unit 12 on a side surface side intersecting a detection surface of the X-ray detection unit 20 at an imaging position in the standing position. The optical imaging unit 31 is provided facing the irradiation direction of X-rays from the X-ray irradiation unit 10. When the X-ray irradiation unit 10 faces toward the subject 101 and the X-ray detection unit 20, the optical imaging unit 31 can capture the optical image 70 of the subject 101 and the X-ray detection unit 20 from the X-ray irradiation unit 10 side. An imaging range of the optical imaging unit 31 is set to include an irradiation field range where X-rays are irradiated and to be larger than the irradiation field range of X-rays.

As shown in FIG. 3, the optical imaging unit 31 includes an imaging element 31a. The optical imaging unit 31 is, for example, an optical camera. The imaging element 31a includes, for example, a CCD (Charge Coupled Device) image sensor or a CMOS (Complementary Metal Oxide Semiconductor) image sensor. The optical imaging control unit 32 is a microcomputer including, for example, a CPU (Central Processing Unit) and a memory. The optical imaging control unit 32 transmits and receives signals to and from the device control unit 60 via a wireless connection or a wired connection.

As shown in FIG. 4, the optical imaging unit 31 is configured to capture the optical image 70 of the subject 101. The optical imaging control unit 32 is configured to receive an input of a signal output by the imaging element 31a and to generate the optical image 70 based on the input signal. The optical imaging control unit 32 also outputs the generated optical image 70 to the display operation unit 41 to display it.

As shown in FIG. 3, the device control unit 60 includes a memory 60a. The device control unit 60 controls X-ray imaging by the X-ray irradiation unit 10 and the X-ray detection unit 20, and controls movement relating to the holding unit 40. Specifically, the device control unit 60 includes a CPU (Central Processing Unit). The input unit 61 has a function of receiving an input operation relating to X-ray imaging. The input operation is setting of imaging conditions of X-ray imaging, an instruction to start X-ray irradiation, and the like. The device control unit 60 controls X-ray imaging based on parameters and various programs preset and stored in the memory 60a. The device control unit 60 controls an adjustment amount of an irradiation field by the collimator unit 12.

In the X-ray imaging system 100, the movement mechanism 50 has a drive unit 50a such as a motor, and an operation detection unit 50b that detects an operation of the drive unit 50a. The operation detection unit 50b includes, for example, a potentiometer that detects rotation of the motor. The drive unit 50a and the operation detection unit 50b are arranged in each part of the movement mechanism 50 that moves the holding unit 40, and are also arranged in each of the imaging table 21 and the imaging stand 22 to change the position of the X-ray detection unit 20. The device control unit 60 changes a position and an angle of the X-ray irradiation unit 10 and a position of the X-ray detection unit 20 by controlling the operation of the movement mechanism 50 based on an input operation to the input unit 61 or the display operation unit 41, or an operation force to the grip unit 42 of the holding unit 40. In addition, the device control unit 60 controls the operation of the movement mechanism 50 by feedback control by outputting a control signal to control the operation of the drive unit 50a and receiving an input of a detection signal indicating the operation of the drive unit 50a detected by the operation detection unit 50b.

In the present embodiment, the device control unit 60 outputs the position and the angle of the X-ray irradiation unit 10 held by the holding unit 40 and the position of the X-ray detection unit 20 arranged on each of the imaging table 21 and the imaging stand 22 to the optical imaging control unit 32. The optical imaging control unit 32 acquires an arrangement of the X-ray irradiation unit 10 and an arrangement of the X-ray detection unit 20 based on the input from the device control unit 60. The optical imaging control unit 32 also acquires the adjustment amount of the irradiation field by the collimator unit 12 based on the input from the device control unit 60.

### (Control of Display by Optical Imaging Control Unit)

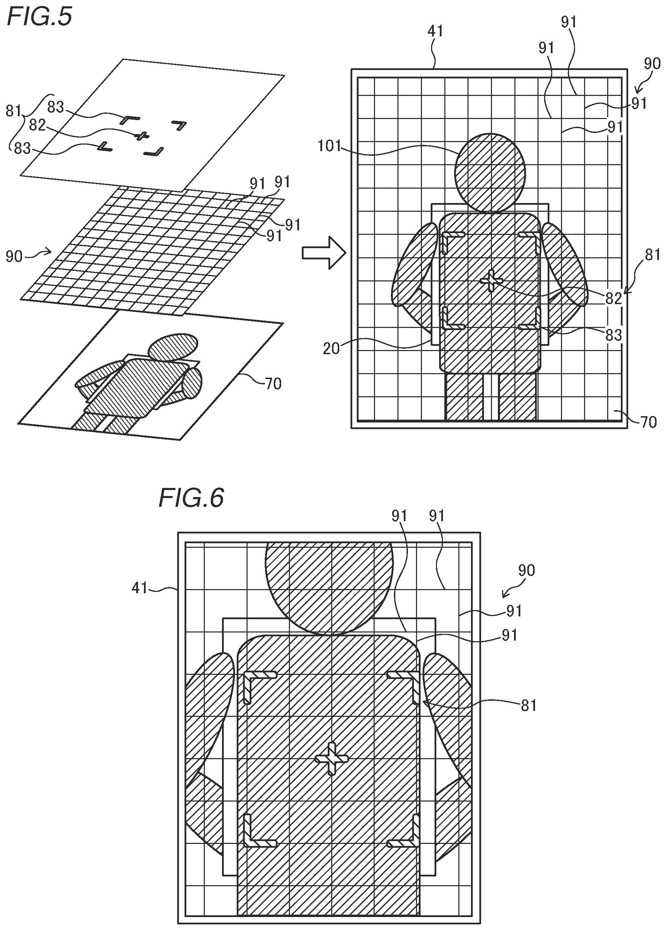

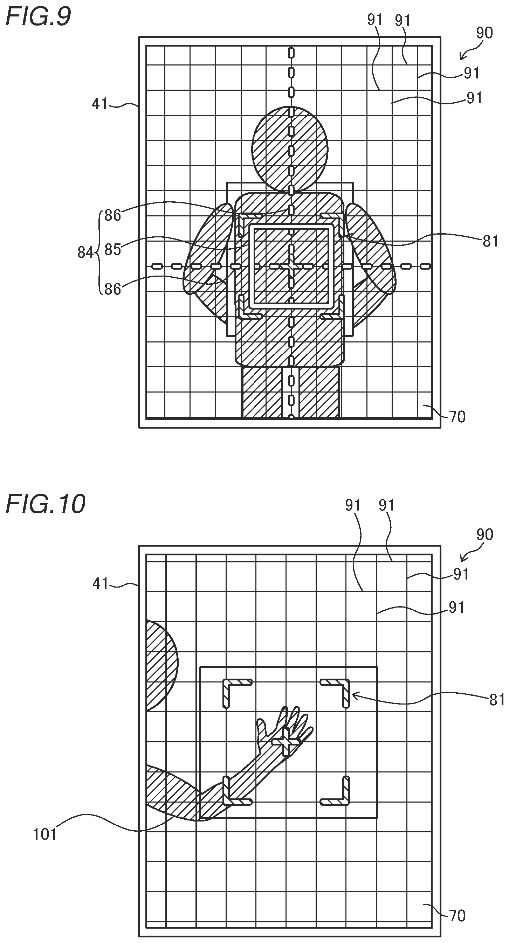

As shown in FIG. 5, in the present embodiment, the optical imaging control unit 32 is configured to cause the display operation unit 41 to superimpose and display a detection unit region display 81 and a position reference image 90 on the optical image 70. In the following description, the control of the display of the display operation unit 41 by the optical imaging control unit 32 in a case where X-ray imaging is performed in a posture in which the subject 101 is standing (standing position) will be described. Further, since the above control is similar in a case where X-ray imaging is performed in a posture in which the subject 101 is lying down on the imaging table 21 (decubitus position), description thereof will be omitted. FIGS. 5 to 9 show an example in which X-ray imaging of a chest portion of the subject 101 is performed, and FIG. 10 shows an example in which X-ray imaging of a palmar part of the subject 101 is performed.

The detection unit region display 81 indicates a region in which the X-ray detection unit 20 is arranged in the optical image 70. The optical imaging control unit 32 acquires a three-dimensional arrangement of the X-ray irradiation unit 10 and the X-ray detection unit 20, and also acquires a three-dimensional arrangement of the optical imaging unit 31. Since the optical imaging unit 31 is arranged in the holding unit 40 together with the X-ray irradiation unit 10, the optical imaging control unit 32 calculates a three-dimensional position of the optical imaging unit 31 using previously stored parameters based on the acquired position of the X-ray irradiation unit 10, for example. Then, the optical imaging control unit 32 performs a geometric calculation based on a three-dimensional positional relationship among the acquired X-ray irradiation unit 10, X-ray detection unit 20, and optical imaging unit 31 to detect a region in which the X-ray detection unit 20 is arranged in the optical image 70. The optical imaging control unit 32 may acquire a position of the optical imaging unit 31 calculated by the device control unit 60.

The optical imaging control unit 32 generates the detection unit region display 81 based on the detected region in which the X-ray detection unit 20 is arranged. In the optical image 70, the region in which the X-ray detection unit 20 is arranged is represented by a rectangular shape. The detection unit region display 81 shows the region in which the X-ray detection unit 20 is arranged in a rectangular shape. The detection unit region display 81 shows, for example, a square region. The detection unit region display 81 has a center display 82 indicating a center of the region in which the X-ray detection unit 20 is arranged, and four region displays 83 indicating four corners of the rectangular region by L-shapes. The center display 82 has a cross shape and is arranged at a position where diagonal lines of the rectangular region intersect. The center display 82 and the four region displays 83 are colored and displayed, for example, in blue. The detection unit region display 81 also shows a guide of a position and a range of an area where X-rays can be detected in the X-ray detection unit 20 in the optical image 70. The region indicated by the detection unit region display 81 may be a smaller range or a larger range than an actual area where X-rays can be detected in the X-ray detection unit 20.

The position reference image 90 is an image arranged inside the X-ray detection unit 20 and indicating a position relative to a center of the X-ray detection unit 20. The "inside the X-ray detection unit 20" referred to here means inside the region in which the X-ray detection unit 20 is arranged in the image. The position reference image 90 is an image indicating a position relative to a center of the region in which the X-ray detection unit 20 is arranged, inside the region in which the X-ray detection unit 20 is arranged in the optical image 70. The position reference image 90 includes a plurality of linear position reference displays 91. In the position reference image 90, the position reference displays 91 indicate a position relative to a center of the detection unit region display 81 arranged inside the detection unit region display 81. The position reference displays 91 include at least a pair of position reference displays 91 arranged at equal intervals relative to the center of the detection unit region display 81.

Specifically, in the position reference image 90, a plurality of linear position reference displays 91 are arranged orthogonal to each other in a grid pattern. That is, in the position reference image 90, the position reference displays 91 are arranged as grid lines. Furthermore, the position reference image 90 is arranged with reference to a direction of each side of a rectangular region of the X-ray detection unit 20 so as to correspond to a region of the X-ray detection unit 20 having a rectangular shape in the optical image 70. Specifically, in the position reference image 90, the position reference displays 91 are arranged along each side of a rectangle of the detection unit region display 81 so as to correspond to the detection unit region display 81 indicating a rectangular shape. That is, the plurality of linear position reference displays 91 in the position reference image 90 are arranged orthogonal to each other along each side of the rectangle of the detection unit region display 81. In other words, a plurality of linear position reference displays 91 arranged in parallel to each other along one direction and a plurality of linear position reference displays 91 arranged in parallel to each other along another direction are arranged to be orthogonal to each other. Furthermore, in each of the one direction and the other direction, a plurality of linear position reference displays 91 are arranged side by side at equal intervals. That is, in the position reference image 90, a plurality of square cells are formed by arranging linear position reference displays 91 in a grid pattern.

Furthermore, in the present embodiment, one of the linear position reference displays 91 arranged in a grid pattern in the position reference image 90 is arranged so as to superimpose on the center of the detection unit region display 81. Then, one of the linear position reference displays 91 arranged in a grid pattern in the position reference image 90 is arranged so as to superimpose on an outer peripheral edge portion of the detection unit region display 81. That is, the optical imaging control unit 32 is configured to cause the display operation unit 41 to superimpose and display the position reference image 90 on the optical image 70 in a state where linear position reference displays 91 arranged side by side are superimposed on the center and the peripheral portion of the detection unit region display 81.

For example, a plurality of linear position reference displays 91 are arranged such that a total of 16 cells, four in a vertical direction as one direction and four in a horizontal direction as the other direction, are respectively formed inside the rectangular detection unit region display 81. An intersection of a pair of position reference displays 91 orthogonal to each other is arranged at a position overlapping with the center display 82 which is the center of the detection unit region display 81. Furthermore, linear position reference displays 91 are arranged coincident with vertical sides and horizontal sides so as to superimpose on boundaries of the rectangular region represented by the detection unit region display 81.

Furthermore, in the present embodiment, the optical imaging control unit 32 is configured to cause the display operation unit 41 to superimpose and display the position reference image 90 on the optical image 70 over an area including an outside of the X-ray detection unit 20. That is, the optical imaging control unit 32 is configured to cause the display operation unit 41 to superimpose and display the position reference image 90 on the optical image 70 over an area including an outside of the detection unit region display 81. Specifically, in the position reference image 90, the position reference displays 91 are arranged over the entire optical image 70. That is, in both the vertical and horizontal directions of the optical image 70, linear position reference displays 91 are arranged so as to extend from one of the peripheral portions to the other.

Furthermore, in the present embodiment, the optical imaging control unit 32 is configured to cause the display operation unit 41 to display the position reference image 90 superimposed on the optical image 70 in a translucent state. For example, in the generated position reference image 90, the optical imaging control unit 32 superimposes the linear position reference displays 91 on the optical image 70 in a translucent state with a transmittance of 50%. In FIG. 5 and FIG. 6 and the subsequent drawings, the position reference display 91 is illustrated in a non-translucent state.

As shown in FIG. 6, the optical imaging control unit 32 is configured to change the size of the position reference image 90 in accordance with the size of the X-ray detection unit 20 in the optical image 70. Specifically, the optical imaging control unit 32 is configured to change an interval between position reference displays 91 included in the position reference image 90 in accordance with the size of the detection unit region display 81 in the optical image 70. When a source to image receptor distance (SID), which is a distance between the X-ray irradiation unit 10 and the X-ray detection unit 20, is changed by the device control unit 60, the optical imaging control unit 32 changes the size of the detection unit region display 81 to be superimposed on the optical image 70 so as to correspond to the changed magnitude of the SID. Then, the optical imaging control unit 32 changes the interval between the position reference displays 91 such that the position reference image 90 is enlarged or reduced corresponding to the changed size of the detection unit region display 81.

The optical imaging control unit 32 changes the interval between the position reference displays 91 so that a ratio of the interval between the position reference displays 91 to the size of the detection unit region display 81 is kept constant. For example, FIG. 6 shows an example where a distance (SID) between the X-ray irradiation unit 10 and the X-ray detection unit 20 is changed to be smaller from a state of FIG. 5. In this case, a size of a region in which the X-ray detection unit 20 is arranged in the optical image 70 becomes larger. The optical imaging control unit 32 largely changes the size of the detection unit region display 81 in accordance with the size of the region where the X-ray detection unit 20 is arranged, and causes the display operation unit 41 to display the position reference image 90 by superimposing it on the optical image 70 in a state where the interval between the position reference displays 91 is made larger.

When the X-ray irradiation unit 10 is translated relative to the detection surface of the X-ray detection unit 20 while keeping the distance (SID) between the X-ray irradiation unit 10 and the X-ray detection unit 20 constant, sizes of the detection unit region display 81 and the position reference image 90 are not changed, and the detection unit region display 81 and the position reference image 90 are translated together with the optical image 70 so that a positional relationship with the optical image 70 becomes a constant state.

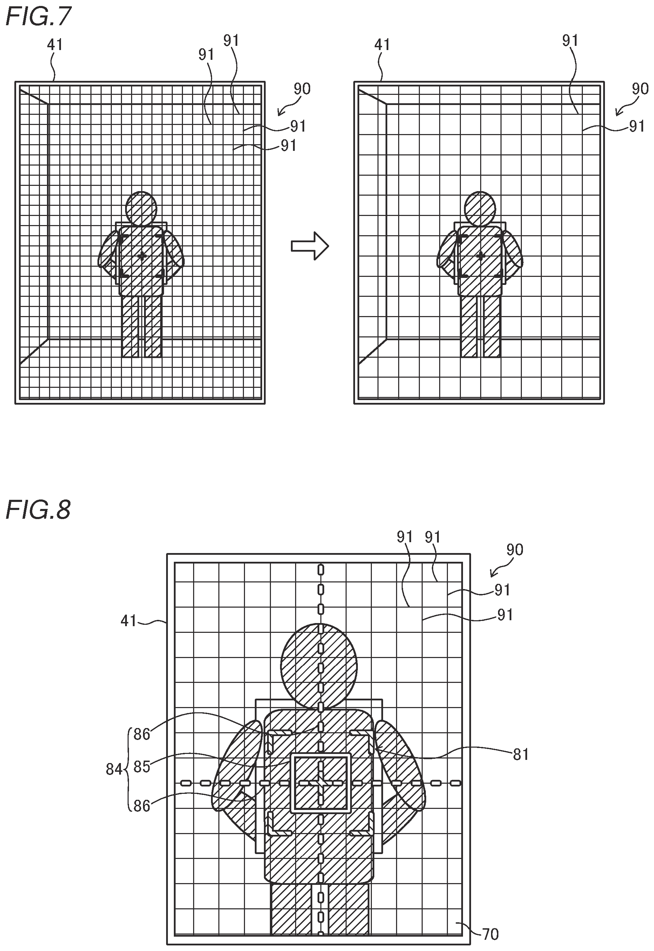

As shown in FIG. 7, the optical imaging control unit 32 is configured to change the number of position reference displays 91 included in the position reference image 90 to be displayed so that the interval between the position reference displays 91 included in the position reference image 90 becomes larger than a predetermined size. For example, FIG. 7 shows an example where the distance (SID) between the X-ray irradiation unit 10 and the X-ray detection unit 20 is changed to be larger from the state of FIG. 5. In this case, since the interval between the position reference displays 91 becomes smaller, the optical imaging control unit 32 decreases the number of position reference displays 91 to be displayed on the display operation unit 41 in the position reference image 90 to thin out. For example, the optical imaging control unit 32 changes the number of displayed position reference displays 91 according to a value of the SID.

As an example, when the value of the SID is smaller than a preset predetermined threshold value, the optical imaging control unit 32 displays a plurality of linear position reference displays 91 so that a total of 16 cells, four each in the vertical direction and the horizontal direction, are formed inside the detection unit region display 81. And, when the value of the SID is equal to or larger than the preset predetermined threshold value, the optical imaging control unit 32 displays a plurality of linear position reference displays 91 so that a total of 4 cells, two each in the vertical direction and the horizontal direction, are formed inside the detection unit region display 81. Even when changing the display number, the optical imaging control unit 32 decreases the position reference displays 91 arranged side by side every other one so that the interval between the position reference displays 91 becomes equal. The optical imaging control unit 32 may increase the number of displayed position reference displays 91 when the value of the SID is changed to be smaller.

As shown in FIG. 8, the optical imaging control unit 32 is configured to cause the display operation unit 41 to superimpose and display an irradiation field region display 84 indicating a region to which X-rays are irradiated by the X-ray irradiation unit 10, together with the detection unit region display 81 and the position reference image 90, on the optical image 70. Similar to the detection unit region display 81, the optical imaging control unit 32 detects a region to which X-rays are irradiated by the X-ray irradiation unit 10 in the optical image 70 by performing a geometric calculation based on the three-dimensional positional relationship among the acquired X-ray irradiation unit 10, X-ray detection unit 20, and optical imaging unit 31. Then, the optical imaging control unit 32 generates an irradiation field region display 84 based on the detected region to which X-rays are irradiated.

Similar to the detection unit region display 81, the irradiation field region display 84 indicates the region to which X-rays are irradiated in a rectangular shape. The irradiation field region display 84 indicates, for example, a square region. The irradiation field region display 84 has a region display 85 that is a rectangular frame line indicating a region of an irradiation field to which X-rays are irradiated, and a dotted line display 86 that is a pair of linear dotted lines perpendicular to each other to indicate a center of the region of the irradiation field to which X-rays are irradiated. The pair of dotted line displays 86 are arranged while intersecting at right angles so as to pass through a center position where diagonal lines of the rectangle of the region display 85 intersect. Furthermore, the pair of dotted line displays 86 are arranged so as to extend over an outside of the region display 85 which is a rectangular frame line. The region display 85 and the pair of dotted line displays 86 are colored differently from the detection unit region display 81, for example, in yellow, and displayed. The irradiation field region display 84 indicates a guide for a position and a range of a region to which X-rays are irradiated in the optical image 70. The region indicated by the irradiation field region display 84 may have a smaller range or a larger range than a region to which X-rays are actually irradiated.

As shown in FIG. 9, the optical imaging control unit 32 is configured to change a size of the irradiation field region display 84 superimposed on the optical image 70 on the display operation unit 41 according to the X-ray irradiation field specified by an adjustment of the collimator unit 12. The optical imaging control unit 32 changes a size of the region display 85 which is a rectangular frame line in the irradiation field region display 84 to be superimposed on the optical image 70 based on a value of the adjustment amount of the collimator unit 12 acquired from the device control unit 60. For example, FIG. 9 shows an example where the adjustment amount of the collimator unit 12 is changed from the state of FIG. 8 so that the region of the X-ray irradiation field becomes larger. In this case, in the irradiation field region display 84, a size of the region display 85 indicated by a rectangular frame line is changed, and a size of the dotted line display 86 is not changed.

The optical imaging control unit 32 may switch whether to display each of the detection unit region display 81, the irradiation field region display 84, and the position reference image 90 on the display operation unit 41 based on an input operation to the display operation unit 41 or an input operation to the input unit 61. For example, the optical imaging control unit 32 switches from a state where the detection unit region display 81 and the position reference image 90 are superimposed and displayed on the optical image 70 to a state where all of the detection unit region display 81, the irradiation field region display 84, and the position reference image 90 are superimposed and displayed on the optical image 70 based on an acceptance of an input operation.

Further, as shown in FIG. 10, also when X-ray imaging of a palmar part of the subject 101 is performed, the optical imaging control unit 32 causes the display operation unit 41 to superimpose and display the detection unit region display 81 and the position reference image 90 on the optical image 70 in a similar manner to the X-ray imaging of the chest portion. The optical imaging control unit 32 causes the display operation unit 41 to superimpose and display the position reference image 90 together with the detection unit region display 81 on the optical image 70 so that an arrangement of the subject 101 in the detection unit region display 81 can be confirmed even when a size of an area targeted for the subject 101 such as the palmar part is smaller than the region where X-rays are detected. The optical imaging control unit 32 may superimpose and display the irradiation field region display 84 on the optical image 70 in which the palmar part is imaged in a similar manner to the X-ray imaging of the chest portion.

In the examples of FIGS. 5 to 10, an example is shown where an irradiation direction of X-rays by the X-ray irradiation unit 10 and the detection surface of the X-ray detection unit 20 are orthogonal to each other. When an irradiation direction of X-rays is inclined without being orthogonal to the detection surface of the X-ray detection unit 20, an inclination of arrangement and a shape of the position reference display 91 in the position reference image 90 may change. The optical imaging control unit 32 may stop displaying the position reference image 90 when at least one of the X-ray irradiation unit 10 and the X-ray detection unit 20 is moved such that an irradiation direction of X-rays is inclined with respect to the detection surface of the X-ray detection unit 20, or may change a display in the position reference image 90 so as to correspond to an arrangement relationship between the X-ray irradiation unit 10 and the X-ray detection unit 20. In addition, whether or not to stop displaying the position reference image 90 may be changed based on an input operation by an operator.

### (Image Display Method)

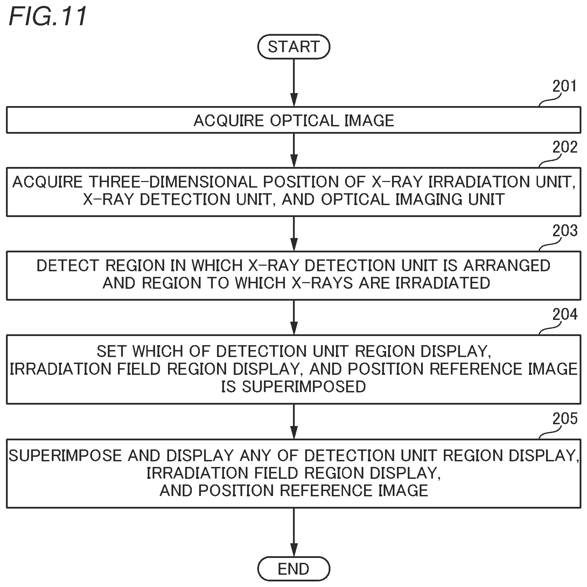

Next, with reference to FIG. 11, control processing of an image display method by the X-ray imaging system 100 according to the present embodiment will be described. Control processing of the image display method in steps 201 to 205 is executed by the optical imaging control unit 32.

First, in step 201, the optical image 70 captured by the optical imaging unit 31 is acquired.

Next, in step 202, a three-dimensional position of the X-ray irradiation unit 10 and the X-ray detection unit 20 is acquired, and a three-dimensional position of the optical imaging unit 31 is calculated and acquired, based on a signal from the device control unit 60.

Next, in step 203, a region in which the X-ray detection unit 20 is arranged in the optical image 70 is detected, and a region to which X-rays are irradiated is detected.

Next, in step 204, it is set which of the detection unit region display 81, the irradiation field region display 84, and the position reference image 90 is superimposed on the optical image 70. For example, based on an input operation on the display operation unit 41, it is set which of the detection unit region display 81, the irradiation field region display 84, and the position reference image 90 is superimposed on the optical image 70.

Next, in step 205, any of the detection unit region display 81, the irradiation field region display 84, and the position reference image 90 set in step 204 is superimposed on the optical image 70 and displayed on the display operation unit 41. For example, the detection unit region display 81 and the position reference image 90 are superimposed on the optical image 70 and displayed on the display operation unit 41.

For example, the optical imaging control unit 32 repeatedly executes the control processing of step 201 to step 205 at every predetermined control cycle. In step 201 and step 202, either control process may be performed first.

### (Effects of this Embodiment)

In the present embodiment, the following effects can be obtained.

In the present embodiment, as described above, an optical imaging control unit 32 (control unit) is provided that is configured to cause a display operation unit 41 (display unit) to superimpose and display a position reference image 90 on an optical image 70, the position reference image 90 being arranged inside an X-ray detection unit 20 and indicating a position relative to a center of the X-ray detection unit 20. Thus, since the position reference image 90 arranged inside the X-ray detection unit 20 and indicating the position relative to the center of the X-ray detection unit 20 is superimposed and displayed on the optical image 70, an operator can confirm a position of a subject 101 inside the X-ray detection unit 20 based on the position reference image 90 by visually recognizing the optical image 70 on which the position reference image 90 is superimposed. As a result, an arrangement of the subject 101 can be confirmed in a region where X-rays are detected.

Furthermore, since the position reference image 90 is superimposed and displayed on the optical image 70, when performing multiple X-ray imaging at predetermined periods to observe changes over time in a target site, it is possible to confirm an arrangement of the subject 101 in a region where X-rays are detected in each of the multiple X-ray imaging. Therefore, in each of multiple X-ray imagings, it is possible to easily perform a positioning of a target site of a subject 101 in a region where X-rays are detected. As a result, when changes over time in the same site of the subject 101 are observed by X-ray imaging, consistency in arrangement of the target site of the subject 101 can be ensured in an X-ray image generated by X-ray imaging.

Furthermore, in the present embodiment, by configuring as described below, the following further effects are obtained.

That is, in the present embodiment, as described above, an optical imaging control unit 32 (control unit) is configured to cause a display operation unit 41 (display unit) to superimpose and display, on an optical image 70, a detection unit region display 81 indicating a region in which an X-ray detection unit 20 is arranged in the optical image 70, and a position reference image 90 including a position reference display 91 arranged inside the detection unit region display 81 and indicating a position relative to a center of the detection unit region display 81. By configuring in this manner, since the position reference image 90 including the position reference display 91 arranged inside the detection unit region display 81 and indicating the position relative to the center of the detection unit region display 81 is superimposed and displayed on the optical image 70, an operator can confirm a position of a subject 101 inside the X-ray detection unit 20 based on the position reference display 91 of the position reference image 90 by visually recognizing the optical image 70 on which the position reference image 90 is superimposed. As a result, an arrangement of the subject 101 can be confirmed in a region where X-rays are detected.

Furthermore, in the present embodiment, as described above, an optical imaging control unit 32 (control unit) is configured to cause a display operation unit 41 (display unit) to superimpose and display, on an optical image 70, a position reference image 90 including at least a pair of position reference displays 91 arranged at equal intervals from a center of a detection unit region display 81. By configuring in this manner, since the position reference displays 91 in the position reference image 90 are arranged at equal intervals relative to the center of the detection unit region display 81, an operator can confirm an arrangement relationship between a center position of the detection unit region display 81 indicating a region in which an X-ray detection unit 20 is arranged and a position of a subject 101. Therefore, an operator can verify a position of a subject 101 inside the detection unit region display 81 in detail based on the position reference display 91 by visually recognizing the optical image 70 on which the position reference image 90 is superimposed. As a result, an arrangement of a subject 101 can be verified in detail in a region where X-rays are detected.

Furthermore, in the present embodiment, as described above, an optical imaging control unit 32 (control unit) is configured to cause a display operation unit 41 (display unit) to superimpose and display, on an optical image 70, a position reference image 90 in which a plurality of linear position reference displays 91 are arranged perpendicular to each other in a grid pattern. By configuring in this manner, it is possible to verify an arrangement relationship between a subject 101 and the linear position reference displays 91 arranged in a grid pattern in the position reference image 90. Therefore, an operator can visually recognize the optical image 70 on which the position reference image 90 is superimposed to confirm in more detail a position of the subject 101 inside a detection unit region display 81 indicating a region where an X-ray detection unit 20 is arranged based on the position reference display 91. As a result, an arrangement of the subject 101 can be confirmed in more detail in a region where X-rays are detected. Additionally, because the position reference display 91 in the position reference image 90 is linear, an operator can easily verify whether or not the subject 101 is inclined in a region where X-rays are detected. For example, when performing X-ray imaging of a chest portion of the subject 101, by visually recognizing the optical image 70 on which the position reference image 90 including linear position reference displays 91 is superimposed, it can be easily verified whether or not the chest portion of the subject 101 is inclined left or right, and thus, it can be easily suppressed that an X-ray image with left and right lungs of different sizes is taken due to the inclination of the chest portion of the subject 101.

Furthermore, in the present embodiment, as described above, an optical imaging control unit 32 (control unit) is configured to cause a display operation unit 41 (display unit) to superimpose and display a position reference image 90 on an optical image 70 in a state where one of linear position reference displays 91 arranged in a grid pattern in the position reference image 90 is arranged so as to superimpose on a center of a detection unit region display 81. By configuring in this manner, since one of the linear position reference displays 91 arranged in a grid pattern in the position reference image 90 is arranged to overlap with the center of the detection unit region display 81, an operator can visually recognize the optical image 70 on which the position reference image 90 is superimposed to verify in more detail an arrangement relationship between a center position of the detection unit region display 81 indicating a region where an X-ray detection unit 20 is arranged and a position of a subject 101 based on the position reference display 91. As a result, an arrangement of the subject 101 can be confirmed in even greater detail in a region where X-rays are detected.

Furthermore, in the present embodiment, as described above, an optical imaging control unit 32 (control unit) is configured to cause a display operation unit 41 (display unit) to superimpose and display a position reference image 90 on an optical image 70 in a state where one of linear position reference displays 91 arranged in a grid pattern in the position reference image 90 is arranged so as to overlap with an outer peripheral edge portion of a detection unit region display 81. By configuring in this manner, it is easier to recognize an arrangement of a subject 101 relative to a base position reference display 91 inside a detection unit region display 81, as compared to a case where a linear position reference display 91 is arranged offset with respect to the outer peripheral edge portion of the detection unit region display 81. As a result, an operator can visually recognize the optical image 70 on which the position reference image 90 is superimposed to verify in even greater detail an arrangement of the subject 101 based on the position reference display 91 in a region where X-rays are detected.

Furthermore, in the present embodiment, as described above, an optical imaging control unit 32 (control unit) is configured to change a size of a position reference image 90 in accordance with a size of an X-ray detection unit 20 in an optical image 70. By configuring in this manner, even when a separation distance between an X-ray irradiation unit 10 and an X-ray detection unit 20 is altered and a size of the X-ray detection unit 20 in the optical image 70 changes, an arrangement relationship of a subject 101 to the position reference image 90 can be prevented from altering by modifying the size of the position reference image 90 in accordance with the size of the X-ray detection unit 20 in the optical image 70. As a result, even if a separation distance between the X-ray irradiation unit 10 and the X-ray detection unit 20 is changed, it is possible to verify an arrangement of the subject 101 in a region where X-rays are detected.

Furthermore, in the present embodiment, as described above, an optical imaging control unit 32 (control unit) is configured to modify a display number of position reference displays 91 included in a position reference image 90 so that an interval between the position reference displays 91 included in the position reference image 90 becomes larger than a predetermined size. By configuring in this manner, it is possible to restrain a reduction in visibility of the optical image 70 on which the position reference image 90 is superimposed owing to an interval between position reference displays 91 becoming excessively narrow. As a result, it is possible to avoid difficulty in confirming an arrangement of a subject 101 in a region where X-rays are detected due to a decrease in the visibility of the optical image 70.

Furthermore, in the present embodiment, as described above, an optical imaging control unit 32 (control unit) is configured to cause a display operation unit 41 (display unit) to superimpose and display, on an optical image 70, a position reference image 90 arranged taking as reference a direction of each side of a rectangular region of an X-ray detection unit 20 so as to correspond to a region of the X-ray detection unit 20 having a rectangular shape in the optical image 70. By configuring in this manner, because the position reference image 90 is arranged referencing an orientation of each side of the rectangle of the region of the X-ray detection unit 20, the position reference image 90 can be arranged matching a shape of a region where X-rays are detected. Therefore, it is possible to verify an arrangement of a subject 101 in conformity with a shape of a region where X-rays are detected.

Furthermore, in the present embodiment, as described above, an optical imaging control unit 32 (control unit) is configured to cause a display operation unit 41 (display unit) to superimpose and display a position reference image 90 on an optical image 70 over an area inclusive of an outside of an X-ray detection unit 20. By configuring in this manner, an operator can check an arrangement of a subject 101 inside a region where X-rays are detected, referring to an arrangement of the subject 101 placed outside the region where X-rays are detected, based on the position reference image 90. Thus, an arrangement of the subject 101 in the region where X-rays are detected can be confirmed in finer detail than when the position reference image 90 is arranged solely within the region where X-rays are detected. For example, when capturing an X-ray image of a chest region of the subject 101, a layout of not only the chest region of the subject 101 positioned inside the region where X-rays are detected, but also an arrangement of shoulders, arms, a head portion and the like of the subject 101 located outside the region where X-rays are detected can be confirmed via the position reference image 90. Therefore, since an arrangement of a chest region of the subject 101 inside a region where X-rays are detected can be verified with reference to the arrangement of the shoulders, arms, head portion and the like of the subject 101, an arrangement of the subject 101 can be verified in more detail compared to a case where the position reference image 90 is placed solely in positions corresponding to a chest region which is an object of an X-ray scan.

Furthermore, in the present embodiment, as described above, an optical imaging control unit 32 (control unit) is configured to cause a display operation unit 41 (display unit) to superimpose and display on an optical image 70 an irradiation field region display 84 denoting a region to which X-rays are irradiated by an X-ray irradiation unit 10, alongside a detection unit region display 81 and a position reference image 90. By configuring in this manner, an operator visually recognizing the optical image 70 over which the position reference image 90, the detection unit region display 81, and the irradiation field region display 84 are superimposed can affirm an arrangement of an irradiation field of X-rays relative to a region where X-rays are detected with reference to position reference displays 91 in the position reference image 90. As a result, an arrangement of an irradiation field of X-rays relative to a region where X-rays are detected can be verified in addition to an arrangement of a subject 101 in the region where X-rays are detected based on the position reference displays 91. Furthermore, when adjusting an irradiation field of X-rays irradiated from an X-ray tube 11 of an X-ray irradiation unit 10 by a collimator unit 12, an operator can easily execute adjustments to the X-ray irradiation field by the collimator unit 12 by visually acknowledging a position reference image 90 and an irradiation field region display 84 superimposed and displayed on the optical image 70.

Furthermore, in the present embodiment, as described above, the optical imaging control unit 32 (control unit) is configured to cause the display operation unit 41 (display unit) to display the position reference image 90 superimposed on the optical image 70 in a translucent state. With this configuration, since the position reference image 90 is superimposed on the optical image 70 in a translucent state, even when the position reference image 90 is superimposed on the optical image 70, a reduction in visibility of the optical image 70 can be suppressed. As a result, it is possible to suppress verification of an arrangement of the subject 101 in a region where X-rays are detected from becoming difficult due to the reduction in visibility of the optical image 70.

Furthermore, in the present embodiment, as described above, an X-ray imaging system 100 is provided with a holding unit 40 holding an X-ray irradiation unit 10. An optical imaging unit 31 is set up at the holding unit 40 together with the X-ray irradiation unit 10. By configuring in this manner, since the X-ray irradiation unit 10 and the optical imaging unit 31 are both located on the holding unit 40, an irradiation direction of X-rays irradiated from the X-ray irradiation unit 10 and an imaging direction of the optical imaging unit 31 can be aligned. Hence, alignment of the X-ray irradiation unit 10 can be performed effortlessly while adverting to an optical image 70 taken by the optical imaging unit 31.

### [Modifications]

The embodiment disclosed herein is to be considered in all respects as illustrative and not restrictive. The scope of the present invention is defined by the claims rather than by the foregoing description, and is intended to include all modifications within the scope and meaning equivalent to the claims.

For example, in the above embodiment, an example has been described in which the optical imaging control unit 32 (control unit) including a microcomputer is configured to cause the display operation unit 41 (display unit) to display the position reference image 90 superimposed on the optical image 70, but the present invention is not limited thereto. In the present invention, a device control unit that controls X-ray imaging may be configured to cause the position reference image to be displayed superimposed on the optical image. Further, the optical image on which the position reference image has been superimposed by the control unit (optical imaging control unit) may be output to the outside, and the position reference image may also be displayed superimposed on the optical image on a display unit arranged at a position separated from the control unit. For example, the optical image on which the position reference image has been superimposed may be displayed on a display unit provided in the device control unit arranged outside the imaging room. Further, the control unit may be configured by a personal computer, a processor, or circuitry. Further, each item of control processing by the control unit may be executed by a combination of different pieces of hardware.

Further, in the above embodiment, an example has been described in which the detection unit region display 81 indicating a region in which the X-ray detection unit 20 is arranged and the position reference image 90 are displayed superimposed on the optical image 70, and the position reference image 90, which is arranged inside the X-ray detection unit 20 and indicates a position relative to a center of the X-ray detection unit 20, includes the position reference display 91 arranged inside the detection unit region display 81 indicating the region in which the X-ray detection unit 20 is arranged and indicating a position relative to a center of the detection unit region display 81, but the present invention is not limited thereto. In the present invention, the position reference display in the position reference image may indicate a position relative to the center of the X-ray detection unit in the optical image, instead of indicating a position relative to the center of the detection unit region display. For example, the detection unit region display indicating the region in which the X-ray detection unit is arranged may not be superimposed on the optical image.

Further, in the above embodiment, an example has been described in which the position reference image 90 including the position reference displays 91 arranged at positions equidistant from each other relative to the center of the detection unit region display 81 is displayed superimposed on the optical image 70, but the present invention is not limited thereto. In the present invention, the position reference displays need not be arranged at positions equidistant from each other relative to the center of the detection unit region display. In the position reference image, a plurality of position reference displays may be arranged at positions equidistant from each other while being offset from the center of the detection unit region display. Further, the position reference displays may be arranged at positions symmetrical with respect to the center of the detection unit region display without being arranged at positions equidistant from each other.

Further, in the above embodiment, an example has been described in which, in the position reference image 90, a plurality of translucent linear position reference displays 91 are arranged in a grid pattern so as to be orthogonal to each other, but the present invention is not limited thereto. In the present invention, the position reference displays may be arranged not as linear displays but as points arranged side by side. For example, the position reference displays may be arranged as a plurality of points representing intersections of a plurality of straight lines arranged in a grid pattern. Further, a plurality of straight lines arranged in parallel with each other without intersecting each other may be used as the position reference displays. Further, the position reference displays may be indicated by dotted lines instead of solid lines. Further, the position reference displays need not be translucent. Further, the position reference displays may be displayed in a flashing manner on the display unit. Further, at least one pair of position reference displays may be arranged in the position reference image. The shape of the position reference displays is not limited to a straight line or a point, and may be another shape such as a rectangle or a circle, or a combination of figures having a plurality of different shapes.

In the above embodiment, an example has been described in which the linear position reference display 91 is arranged so as to overlap the center and the peripheral edge portion of the detection unit region display 81, but the present invention is not limited thereto. In the present invention, the linear position reference display may be arranged at a position that does not overlap the center of the detection unit region display. For example, the center of each grid cell formed by grid-like position reference displays orthogonal to each other may overlap the center of the detection unit region display. Further, the position reference display may be arranged so as not to overlap the peripheral edge portion of the detection unit region display.

Further, in the above embodiment, an example has been described in which the position reference displays 91 are arranged along respective sides of a rectangle of the detection unit region display 81 so as to correspond to the detection unit region display 81, and an interval between the position reference displays 91 is changed according to the size of the detection unit region display 81, but the present invention is not limited thereto. In the present invention, the position reference displays may be arranged regardless of the size, position, and orientation of the detection unit region display. For example, a position reference image in which the position reference displays are arranged in a fixed arrangement in a display area of the optical image may be displayed. That is, even when the position and size of the X-ray detection unit in the optical image are changed, the position reference image may remain unchanged. Further, the position reference displays may be arranged along respective sides of the rectangle of the detection unit region display, and an interval between the position reference displays may be kept unchanged even when the size of the detection unit region display is changed.

Further, in the above embodiment, an example has been described in which, when an interval between the position reference displays 91 is changed, the number of displayed position reference displays 91 is changed according to the value of SID, but the present invention is not limited thereto. In the present invention, the number of displayed position reference displays may be changed based on a magnitude of an interval between the position reference displays in the position reference image, instead of the value of SID. Further, the number of displayed position reference displays included in the position reference image may be controlled so as not to exceed a predetermined threshold. The number of displayed position reference displays may be changed based on a ratio of an interval between the position reference displays to the display area of the optical image. Further, the number of displayed position reference displays may remain unchanged.

Further, in the above embodiment, an example has been described in which the position reference image 90 is displayed superimposed on the optical image 70 over an area including an area outside the X-ray detection unit 20, but the present invention is not limited thereto. In the present invention, the position reference image may be displayed only inside the area of the X-ray detection unit. Further, even when the position reference image is displayed over an area including an area outside the X-ray detection unit, the position reference image may be displayed over only a part of the optical image instead of the entirety of the optical image.

Further, in the above embodiment, an example has been described in which the detection unit region display 81, the irradiation field region display 84, and the position reference image 90 are displayed superimposed on the optical image 70, but the present invention is not limited thereto. In the present invention, the irradiation field region display may be omitted from being displayed superimposed on the optical image. For example, an illumination unit indicating a region to which X-rays are irradiated by projecting illumination light onto the subject may be provided.

Further, in the above embodiment, an example has been described in which the imaging unit 30 including the optical imaging unit 31 and the optical imaging control unit 32 (control unit) is arranged in the holding unit 40 that holds the X-ray irradiation unit 10, but the present invention is not limited thereto. In the present invention, the optical imaging unit and the control unit may be arranged at positions separated from each other. Further, the optical imaging unit may be arranged at a position different from the holding unit, such as a ceiling or a wall surface of an imaging room. Further, the imaging unit may be configured as a retrofit unit for an X-ray imaging apparatus.

Further, in the above embodiment, an example has been described in which the position and angle of the X-ray irradiation unit 10 and the position of the X-ray detection unit 20 are acquired based on a detection signal from the operation detection unit 50b in the movement mechanism 50 that moves the X-ray irradiation unit 10 and the X-ray detection unit 20, but the present invention is not limited thereto. In the present invention, a relative positional relationship between the X-ray detection unit and the X-ray irradiation unit may be acquired by arranging a detection unit such as a magnetic sensor in at least one of the X-ray detection unit and the X-ray irradiation unit. Further, a region in which the X-ray detection unit is arranged in the optical image may be detected by performing image processing on the optical image. For example, a marker member indicating a position of the X-ray detection unit may be arranged together with the X-ray detection unit, and the region in which the X-ray detection unit is arranged may be detected by detecting the marker member from the captured optical image.

Also,
Further, in the above embodiment, an example has been described in which the detection unit region display 81 includes a cross-shaped center display 82 and four region displays 83 indicating four corners, and the irradiation field region display 84 includes a region display 85 in the form of a rectangular frame line and a pair of dotted-line displays 86, but the present invention is not limited thereto. In the present invention, the detection unit region display and the irradiation field region display may be displayed in a mode different from that of the above embodiment. For example, either the detection unit region display or the irradiation field region display may be configured not to include a display indicating a center of a region. Further, in the detection unit region display, a range of a region may be indicated by a rectangular frame line. In the irradiation field region display, a range of a rectangular region may be indicated only by displays indicating four corners. At least one of the detection unit region display and the irradiation field region display may be displayed translucently or in a flashing manner.

### [Aspects]

It will be understood by those skilled in the art that the above-described exemplary embodiments are specific examples of the following aspects.

### (Item 1)

An X-ray imaging system comprising:
an X-ray irradiation unit including an X-ray tube;
an X-ray detection unit configured to detect X-rays irradiated from the X-ray irradiation unit and transmitted through a subject;
an optical imaging unit configured to capture an optical image of the subject;
a display unit configured to display the optical image captured by the optical imaging unit; and
a control unit configured to cause the display unit to display, superimposed on the optical image, a position reference image arranged inside the X-ray detection unit and indicating a position relative to a center of the X-ray detection unit, on the optical image.

### (Item 2)

The X-ray imaging system according to item 1, wherein the control unit is configured to cause the display unit to superimpose and display on the optical image: a detection unit region display indicating a region of the optical image in which the X-ray detection unit is arranged in the optical image; and the position reference image including a position reference display arranged inside the detection unit region display and indicating a position relative to a center of the detection unit region display.

### (Item 3)

The X-ray imaging system according to item 2, wherein the control unit is configured to cause the display unit to display, superimposed on the optical image, the position reference image including at least a pair of position reference displays arranged at equal intervals relative to the center of the detection unit region display.

### (Item 4)

The X-ray imaging system according to item 2 or 3, wherein the control unit is configured to cause the display unit to display, superimposed on the optical image, the position reference image including a plurality of linear position reference displays arranged orthogonally to each other in a grid pattern.

### (Item 5)

The X-ray imaging system according to item 4, wherein the control unit is configured to cause the display unit to display, superimposed on the optical image, the position reference image such that one of the linear position reference displays arranged in a grid pattern in the position reference image passes through the center of the detection unit region display.

### (Item 6)

The X-ray imaging system according to item 4 or 5, wherein the control unit is configured to cause the display unit to display, superimposed on the optical image, the position reference image such that one of the linear position reference displays arranged in a grid pattern in the position reference image coincides with an outer peripheral edge portion of the detection unit region display.

### (Item 7)

The X-ray imaging system according to any one of items 1 to 6, wherein the control unit is configured to change a size of the position reference image according to a size of the X-ray detection unit in the optical image.

### (Item 8)

The X-ray imaging system according to any one of items 2 to 6, wherein the control unit is configured to change the number of position reference displays included in the displayed position reference image so that an interval between the position reference displays becomes larger than a predetermined size.

### (Item 9)

The X-ray imaging system according to any one of items 1 to 8, wherein the control unit is configured to cause the display unit to display, superimposed on the optical image, the position reference image arranged with reference to directions of respective sides of a rectangular region of the X-ray detection unit so as to correspond to the rectangular region of the X-ray detection unit in the optical image.

### (Item 10)

The X-ray imaging system according to any one of items 1 to 9, wherein the control unit is configured to cause the display unit to display, superimposed on the optical image, the position reference image over an area including an outside of the X-ray detection unit.

### (Item 11)

The X-ray imaging system according to any one of items 2 to 6, wherein the control unit is configured to cause the display unit to display, superimposed on the optical image, an irradiation field region display indicating a region to which X-rays are irradiated by the X-ray irradiation unit, together with the detection unit region display and the position reference image.

### (Item 12)

The X-ray imaging system according to any one of items 1 to 11, wherein the control unit is configured to cause the display unit to display, superimposed on the optical image, the position reference image in a translucent state.

### (Item 13)

The X-ray imaging system according to any one of items 1 to 12, further comprising a holding unit that holds the X-ray irradiation unit, wherein the optical imaging unit is provided in the holding unit together with the X-ray irradiation unit.

### (Item 14)

An imaging unit comprising:
an optical imaging unit configured to capture an optical image of a subject; and a control unit configured to cause a display unit to display, superimposed on the optical image, a position reference image arranged, in the optical image, inside an X-ray detection unit configured to detect X-rays irradiated from an X-ray irradiation unit and transmitted through the subject, and indicating a position relative to a center of the X-ray detection unit.

### [Explanation of Reference Numerals]

10 X-ray irradiation unit
11 X-ray tube
20 X-ray detection unit
30 Imaging unit
31 Optical imaging unit
32 Optical imaging control unit (control unit)
40 Holding unit
41 Display operation unit (display unit)
70 Optical image
81 Detection unit region display
84 Irradiation field region display
90 Position reference image
91 Position reference display
100 X-ray imaging system
101 Subject

## Claims

1. An X-ray imaging system comprising:
an X-ray irradiation unit including an X-ray tube;
an X-ray detection unit configured to detect X-rays irradiated from the X-ray irradiation unit and transmitted through a subject;
an optical imaging unit configured to capture an optical image of the subject;
a display unit configured to display the optical image captured by the optical imaging unit; and
a control unit configured to cause the display unit to display, superimposed on the optical image, a position reference image arranged inside the X-ray detection unit and indicating a position relative to a center of the X-ray detection unit, on the optical image.

2. The X-ray imaging system according to claim 1, wherein the control unit is configured to cause the display unit to superimpose and display on the optical image: a detection unit region display indicating a region of the optical image in which the X-ray detection unit is arranged in the optical image; and the position reference image including a position reference display arranged inside the detection unit region display and indicating a position relative to a center of the detection unit region display.

3. The X-ray imaging system according to claim 2, wherein the control unit is configured to cause the display unit to display, superimposed on the optical image, the position reference image including at least a pair of position reference displays arranged at equal intervals relative to the center of the detection unit region display.

4. The X-ray imaging system according to claim 2, wherein the control unit is configured to cause the display unit to display, superimposed on the optical image, the position reference image including a plurality of linear position reference displays arranged orthogonally to each other in a grid pattern.

5. The X-ray imaging system according to claim 4, wherein the control unit is configured to cause the display unit to display, superimposed on the optical image, the position reference image such that one of the linear position reference displays arranged in a grid pattern in the position reference image passes through the center of the detection unit region display.

6. The X-ray imaging system according to claim 4, wherein the control unit is configured to cause the display unit to display, superimposed on the optical image, the position reference image such that one of the linear position reference displays arranged in a grid pattern in the position reference image coincides with an outer peripheral edge portion of the detection unit region display.

7. The X-ray imaging system according to claim 1, wherein the control unit is configured to change a size of the position reference image according to a size of the X-ray detection unit in the optical image.

8. The X-ray imaging system according to claim 2, wherein the control unit is configured to change the number of position reference displays included in the displayed position reference image so that an interval between the position reference displays becomes larger than a predetermined size.

9. The X-ray imaging system according to claim 1, wherein the control unit is configured to cause the display unit to display, superimposed on the optical image, the position reference image arranged with reference to directions of respective sides of a rectangular region of the X-ray detection unit so as to correspond to the rectangular region of the X-ray detection unit in the optical image.

10. The X-ray imaging system according to claim 1, wherein the control unit is configured to cause the display unit to display, superimposed on the optical image, the position reference image over an area including an outside of the X-ray detection unit.

11. The X-ray imaging system according to claim 2, wherein the control unit is configured to cause the display unit to display, superimposed on the optical image, an irradiation field region display indicating a region to which X-rays are irradiated by the X-ray irradiation unit, together with the detection unit region display and the position reference image.

12. The X-ray imaging system according to claim 1, wherein the control unit is configured to cause the display unit to display, superimposed on the optical image, the position reference image in a translucent state.

13. The X-ray imaging system according to claim 1, further comprising a holding unit that holds the X-ray irradiation unit, wherein the optical imaging unit is provided in the holding unit together with the X-ray irradiation unit.

14. An imaging unit comprising:
an optical imaging unit configured to capture an optical image of a subject; and a control unit configured to cause a display unit to display, superimposed on the optical image, a position reference image arranged, in the optical image, inside an X-ray detection unit configured to detect X-rays irradiated from an X-ray irradiation unit and transmitted through the subject, and indicating a position relative to a center of the X-ray detection unit.
